(19) Europäisches Patentamt / European Patent Office / Office européen des brevets

(11) **EP 1 774 021 B1**

(12) **EUROPÄISCHE PATENTSCHRIFT**

(45) Veröffentlichungstag und Bekanntmachung des Hinweises auf die Patenterteilung:
**16.10.2013 Patentblatt 2013/42**

(21) Anmeldenummer: **05779466.1**

(22) Anmeldetag: **01.08.2005**

(51) Int Cl.:
***C12Q 1/02*** *(2006.01)*

(86) Internationale Anmeldenummer:
**PCT/EP2005/008318**

(87) Internationale Veröffentlichungsnummer:
**WO 2006/013078 (09.02.2006 Gazette 2006/06)**

(54) **VERFAHREN UND TEILESATZ FÜR DIE MIKROBIOLOGISCHE BESTIMMUNG VON VITAMINEN IN STOFFGEMISCHEN**

METHOD AND KIT FOR MICROBIOLOGICALLY IDENTIFYING VITAMINS IN SUBSTANCE MIXTURES

PROCEDE ET JEU DE COMPOSANTS POUR DETERMINER PAR VOIE MICROBIOLOGIQUE LA PRESENCE DE VITAMINES DANS DES MELANGES DE SUBSTANCES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **30.07.2004 DE 102004037062**
**25.01.2005 DE 102005003457**

(43) Veröffentlichungstag der Anmeldung:
**18.04.2007 Patentblatt 2007/16**

(73) Patentinhaber: **IFP Privates Institut für Produktqualität GmbH**
**12247 Berlin (DE)**

(72) Erfinder:
• **ARMBRUSTER, Franz Paul**
**64625 Bensheim (DE)**
• **WEBER, Wolfgang**
**IfP Privates Institut für**
**12247 Berlin (DE)**

(74) Vertreter: **Benedum, Ulrich Max**
**Haseltine Lake LLP**
**Theatinerstrasse 3**
**80333 München (DE)**

(56) Entgegenhaltungen:
• **ZAYED GABER ET AL: "Influence of trehalose and moisture content on survival of Lactobacillus salivarius subjected to freeze-drying and storage" PROCESS BIOCHEMISTRY, Bd. 39, Nr. 9, 31. Mai 2004 (2004-05-31), Seiten 1081-1086, XP002370238 ISSN: 1359-5113**
• **KELLEHER B P ET AL: "CRYO-PRESERVATION OF LACTOBACILLUS-LEICHMANNII FOR VITAMIN B-12 MICROBIOLOGICAL ASSAY" MEDICAL LABORATORY SCIENCES, Bd. 47, Nr. 2, 1990, Seiten 90-96, XP009062578 ISSN: 0308-3616**
• **STAPERT E M ET AL: "The effect of freezing and thawing on cellular function in Lactobacillus leichmannii." CRYOBIOLOGY. 1968 JAN-FEB, Bd. 4, Nr. 4, Januar 1968 (1968-01), Seiten 174-176, XP009062586 ISSN: 0011-2240**
• **DE VALDÉZ G F ET AL: "Protective effect of adonitol on lactic acid bacteria subjected to freeze-drying." APPLIED AND ENVIRONMENTAL MICROBIOLOGY. JAN 1983, Bd. 45, Nr. 1, Januar 1983 (1983-01), Seiten 302-304, XP002370240 ISSN: 0099-2240**
• **PATENT ABSTRACTS OF JAPAN Bd. 004, Nr. 081 (C-014), 11. Juni 1980 (1980-06-11) & JP 55 045383 A (AJINOMOTO CO INC), 31. März 1980 (1980-03-31)**
• **PATENT ABSTRACTS OF JAPAN Bd. 016, Nr. 575 (C-1011), 15. Dezember 1992 (1992-12-15) & JP 04 228096 A (SS PHARMACEUT CO LTD), 18. August 1992 (1992-08-18)**

**Beschreibung**

GEBIET DER ERFINDUNG

Die Erfindung betrifft ein Verfahren und einen Teilesatz für die quantitative Bestimmung von lebenswichtigen Substanzen in Stoffgemischen im Allgemeinen und insbesondere in Nahrungs- und Futtermitteln, Kosmetika, Medikamenten, pharmazeutischen Erzeugnissen, Körperflüssigkeiten, medizinischen und sonstigen analytischen Proben.

HINTERGRUND DER ERFINDUNG

Unter Vitaminen versteht man lebensnotwendige Substanzen, die der Organismus über die Nahrung aufnehmen muss. Bei ausgewogener Ernährung ist der tägliche Vitaminbedarf des Menschen völlig gedeckt. Bei Unter- oder Fehlernährung oder einer Resorptionsstörung kann ein Vitaminmangel auftreten und zu Krankheiten führen wie Skorbut, Beriberi, Nachtblindheit und sogar Tod. Nahrungsersatzprodukte wie Sojamilch für Säuglinge werden daher mit Vitaminen ergänzt. Es werden auch sonst Nahrungsmitteln mit Vitaminen versetzt. Die biologische Aktivität der Vitaminen ist strukturabhängig; sie wird in Gewichtseinheiten oder Internationalen Einheiten (IE oder EU) angegeben. Der Vitamingehalt einer Probe lässt sich aber nur schwer und zeitaufwendig bestimmen. Dies gilt besonders für die wasserlöslichen Vitamine und die Spurenvitamine $B_{12}$, Folsäure und Biotin.

Den Vitamingehalt in einem Stoffgemisch kann man bestimmen (i) durch chemisch-physikalische Verfahren, z.B. durch Hochdruckflüssigkeitschromatographie (HPLC). Diese sind für Spurenvitamine wie Vitamin $B_{12}$, Folsäure und Biotin zu wenig empfindlich. (ii) Mit immunologischen Verfahren. Sie sind für viele Bestimmungen wegen Matrixeffekten nicht präzise genug sind und müssen auf jede Probenmatrix adaptiert werden. Dabei treten leicht Interferenzen mit anderen Matrixkomponenten auf, besonders bei Säuglingsnahrung, Katzenfutter, Serum und Blut. (iii) Ferner kann man den Vitamingehalt im Tierversuch bestimmen, was nicht praxisrelevant ist, und (iv) durch mikrobiologische Verfahren. Hierbei wird ein Mikroorganismus, für den das zu bestimmende Vitamin unentbehrlich ist, in einer spezifisch defizienten Nährlösung, versetzt mit Probe oder Vitaminstandard, gezüchtet und dessen Wachstum beziehungsweise Stoffwechsel in Abständen gemessen, zum Beispiel titrimetrisch, gravimetrisch, turbidimetrisch  oder nephelometrisch. Zum Hintergrund der mikrobiologischen Bestimmung seien weiterhin folgende Veröffentlichungen genannt: GORIN,G. et al, Appl. Microbiol., 1970, 20, 641-642; KELLEHER,BP et al., J. Clin. Pathol., 1991, 44(7), 592-595; BUI,M.H., J. Vitam. Nutr. Res., 1999, 69(5), 362-366; MOLOY,A.M. et al., Methods Enzymol., 1997, 281, 43-53; CONRAD,PB et al, Cryobiology, 2000, 41, 17-24. Bei der mikrobiologischen Bestimmung muss man mehrere Verdünnungsreihen anlegen, so dass am Ende der Inkubationszeit ein Wachstums- oder Umsatzwert in den Messbereich der parallelen Standard-Konzentrationsreihe fällt. Für jeden Versuch ist eine nur für diesen Ansatz gültige Standardkurve anzufertigen. Zudem ist aus Sicherheits- und Genauigkeitsgründen jede Konzentrationsstufe der Standard- und der Probenreihe mindestens dreifach anzusetzen. Der Vitamingehalt der Probe wird dann durch Vergleich mit den bekannten Vitamingehalten der parallelen Standardreihe ermittelt. Allgemein gültige Präzisionsangaben sind nicht möglich; der Variationskoeffizient sollte aber bei ungefähr 10 Prozent oder darunter liegen. Die Keimsuspension für die Beimpfung der Standard- und Probenreihe ist nicht lagerfähig und muss für jeden Wachstumsversuch neu angezüchtet werden. Es besteht daher stets die Unsicherheit, ob die Impfsuspension richtig angezüchtet wurde beziehungsweise der Keim die gewünschte Sensitivität und Spezifität hat. Die mikrobiologische Bestimmung von Vitaminen ist sehr arbeits- und zeitaufwendig, und sie bedarf einer erheblichen Labororganisation. Nur wenige Labore im Lebensmittelbereich haben sich auf diese Art der Analytik spezialisiert. Bei den Laborärzten in der Humandiagnostik wurde diese Methode wegen des großen Aufwands aufgegeben, obwohl bis heute keine gleichwertigen Verfahren zur Erfassung des biologisch aktiven Vitamingehalts zur Verfügung stehen. Die mikrobiologische Bestimmung ist aber nach wie vor das Referenzverfahren für alle anderen Verfahren.

ZUSAMMENFASSUNG DER ERFINDUNG

Es ist Aufgabe der Erfindung, ein verbessertes Verfahren und einen Teilesatz für die mikrobiologische Bestimmung von Vitaminen, Aminosäuren, insbesondere Lysin, Methionin oder Cystin, und anderen lebenswichtigen Substanzen wie Cholin oder Inositol zur Verfügung zu stellen, welches die Nachteile des Stands der Technik nicht aufweist und prinzipiell automatenfähig ist.

Diese Aufgabe wird gelöst durch einVerfahren zur quantitativen mikrobiologischen Bestimmung von einzelnen Vitaminen, Aminosäuren oder anderen lebensnotwendigen Stoffen in einem Stoffgemisch, bei dem in dem Kulturgefäß für die mikrobiologische Wachstums- und Stoffwechselbestimmung eine vorbestimmte Zahl vitaler Zellen eines geeigneten Mikroorganismus nach Zusatz von konservierenden Zuckern, Schockgefrieren und Gefriertrocknung trocken gelagert werden. Die Zellen werden hierzu in einer Einfrierlösung, enthaltend zudem 200 bis 500 mmol/L Trehalose, bevorzugt 200 bis 250 mmol/L Trehalose, schockgefroren und gefriergetrocknet werden. Die Einfrierlösung ist bevorzugt das Testmedium für die mikrobiologische Bestimmung. Die Zellen werden in dem Verfahren bei einer Temperatur zwischen -10 und -100°C, bevorzugt zwischen - 18 und -80°C schockgefroren. Das erfindungsgemäße Testbesteck beziehungsweise Teilesatz umfasst derart haltbar gemachte Mikroorganismen in vorgegebener Zahl in ein oder mehreren Kulturgefäßen für die Wachstumsversuche bei der mikrobiologischen Bestimmungen. Die Kulturgefäße sind bevorzugt die Kavitäten einer Mikrotiterplatte.

Das erfindungsgemäße Verfahren zeichnet sich dadurch aus, dass eine exakte Zahl von Zellen eines geeigneten Mikroorganismus in einem Untersuchungsgefäß in Gegenwart eines nicht-reduzierenden konservierenden Zuckers und eines assayspezifischen Nährmediums so konserviert werden, dass die Zellen für längere Zeiträume bei Raumtemperatur gelagert werden können und dass alle Zellen nach Zugabe der Flüssigkeit homogen weiterwachsen. Die Trocknung und Haltbarmachung erfolgt mit geringen Abwandlungen je nach Organismus durch eine Gefriertrocknung, wobei die Keime bei -80°C in einem kleinen Volumen, bevorzugt 0,5 bis 100 Mikroliter, besonders bevorzugt 1 bis 10 Mikroliter, schockgefroren und dann gefriergetrocknet werden. Der nicht-reduzierende konservierende Zucker ist bevorzugt Trehalose. Es können auch Saccharose (*Sucrose*) und Einfachzucker wie Dextrose zugesetzt werden, um Trocknungsschäden des Mikroorganismus zu begegnen. Die bevorzugt eingesetzten Konzentrationen sind 200 bis 500 mmol/L Trehalose (mit oder ohne Sucrose), bevorzugt 200 bis 350 mmol/L Trehalose, besonders bevorzugt 200 bis 250 mmol/L Trehalose. Die Trehalosekonzentration sollte mindestens etwa 200 mmol/L sein. Der Zusatz von zweiwertigen Ionen wie $Ca^{2+}$ beim Trocknen verbessert den Wachstumsstart. Um zu Vermeiden, dass die Mikroorganismen bei der Gefriertrocknung und der Trockenlagerung ihren Stoffwechsel auf eine Mangelumgebung umstellen, werden sie bevorzugt im späteren Assaymedium, natürlich in Abwesenheit der Bestimmungssubstanz, schockgefroren und gefriergetrocknet. Hierdurch wird vermieden, dass während der Gefriertrocknung und der Trockenlagerung Keime generiert werden, die ohne die Bestimmungssubstanz, das Vitamin, wachsen können. Derartige Keime bilden sich ansonsten regelmäßig, führen zu einem hohen Messhintergrund und vereinzelt zu falschen Ergebnissen.

Der Teilesatz ergibt sich als Zwischenerzeugnis aus dem erfindungsgemäßen Verfahren. Der Untersuchungskit für die mikrobiologische Bestimmung von Vitaminen umfasst vorbereitete Reagenzien wie kalibrierte Standards und Nährlösungen für das Ansetzen der Wachstumskurven. Dem Fachmann ist bekannt, dass der Stoffwechsel von Mikroorganismen über Stoffwechselendprodukte wie Ethanol oder Milchsäure bestimmt werden kann. Der Untersuchungskit enthält dann Reagenzien für den Nachweis dieser Stoffwechselendprodukte, beispielsweise über eine geeignete Farbreaktion. Elegant kann das Wachstum der Bakterien auch chemoluminimetrisch bestimmt werden über den ATP-Gehalt nach Aufschluss der Bakterienwand und durch Zusatz von Luciferin und Luciferase.

Das erfindungsgemäße Verfahren ist prinzipiell für die mikrobiologische Gehaltsbestimmung aller Vitamine, Vitaminvorläufer, Vitaminabkömmlingen, Aminosäuren und sonstiger lebenswichtigen Substanzen geeignet. Insbesondere empfiehlt es sich für den Nachweis von Spurenvitaminen. Als Beispiele seien genannt:

*Keim für den Nachweis*

In Klammern sind die jeweils wichtigsten biologischen Vitamin-Wirkstoffe angegeben.

| | |
|---|---|
| Vitamin $B_{12}$ (Cyanocobalamin, Hydroxycobalamin) | *Lactobacillus delbrueckii subsp .lactis (L. leichmanii) ATCC 7830* |
| Folsäure (Folsäure, Pteroylglutaminsäure Folsäurekonjugate) | *Enterococcus hirae ATTC 8043, Lactobacillus rhamnosus ATCC 7469* |
| Biotin | *Lactobacillus plantarum ATCC 8014* |
| Pantothensäure (Pantothensäure, Panthenol) | *Lactobacillus plantarum ATTC 8014* |
| Niacin (Nicotinsäure, Nicotinsäureamid) | *Lactobacillus plantarum* |
| Vitamin $B_1$ (Thiamin, Thiaminpyrophosphat) | *Lactobacillus fermentus ATCC 9338, Weisella (Lactobacillus) viridescens ATCC 12706* |
| Vitamin $B_2$ (Riboflavin, Riboflavinphosphat) | *Lactobacillus rhamnosus ATCC 7469 Lactobacillus helveticus* |
| Vitamin $B_6$ (Pyridoxin, Pyridoxal, Pyridoxamin, Pyridoxal-5'-phosphat) | *Saccharomyces cerevisiae ATCC 9080 Saccharomyces faecalis* |
| Cholin | *Neurospora crassa ATCC 9277* |
| Inositol | *Saccharomyces cerevisiae ATCC 9080* |
| Lysin, Methionin, Cystin | *Pediococcus acidilactici ATCC 8042* |

Ein besonderer Vorteil der erfindungsgemäßen Vitaminbestimmung ist, dass neben der Arbeits- und Zeitersparnis auch alle biologisch gleichwirkenden Verbindungen bestimmt werden. Dies hat für die Bereiche Lebensmittel und Humandiagnostik viele Vorteile:

Lebensmittelbereich, am Beispiel Nicotinsäure und Nicotinsäureamid. Beide Formen sind gleichermaßen vitaminaktiv. Deklariert wird in der Regel nur Niacin, auch wenn beide Formen des Vitamins zugegen sind. Die mikrobiologische Bestimmung erfasst beide Verbindungen.

Humandiagnostik: Auch hier ist die mikrobiologische Analyse im Vorteil. Bis heute werden Serumanalysen falsch interpretiert, da die herkömmlichen Verfahren nur einzelne Formen eines Vitamins erfassen. Die mikrobiologische Analyse liefert den wahren biologisch aktiven Vitamingehalt.

Es ist überraschend, dass das erfindungsgemäße Verfahren reproduzierbare Wachstumskurven mit Standard und Probe ergibt. Zwar werden Mikroorganismen seit Jahrzehnten in Glycerin- und anderen Gefrierlösung oder in flüssigem Stickstoff aufbewahrt. Auch die Lyophilisierung und die Trocknung von Keimen und Sporen war bekannt. Der Stand der Technik lehrt zudem, prokaryotische Mikroorganismen oder Viren in Gegenwart von Trehalose und divalenten Kationen zu trocknen und aufzubewahren, z.B. in den Vertiefungen einer Mikrotiterplatte, wenn eine geschlossene Kühlkette vermieden werden soll (siehe US-Patente 6 610 531; 5 149 653). Ziel dieser Aufbewahrungsverfahren ist aber die prinzipielle Lebensfähigkeit der Keime und der Erhalt der genetischen und immunologischen Identität. Andererseits war auch bekannt, dass Mikroorganismen nach längerer Lagerung, zum Beispiel in einer Einfrierlösung, unterschiedlich lang brauchen, bis Stoffwechsel und Wachstum einsetzen. Werden die Mikroorganismen nach dem Einfrieren in eine Kulturlösung überführt, dann wird in der Regel der Keim, der sich als schnellstes regeniert und als erstes zu wachsen beginnt, alle anderen Keime in der Lösung überwachen. Mit dem schnellstwachsenden Keim wird ein Organismus präferiert, der zu den normal wachsenden verschieden ist. Bislang ging man zudem davon aus, dass jede Form einer Dauerlagerung zu unterschiedlich langen Wachstumsverzögerungen bei der Rekonstitution führt. Die Kombination von mikrobiologischer Vitaminbestimmung und der Gefriertrocknung einer exakten Zahl von Keimen in Gegenwart eines konservierenden Zuckers wie Trehalose in einem Kulturgefäß wie der Kavität einer Mikrotiterplatte oder einem Eppendorfgefäß war nicht bekannt. Es war auch nicht bekannt, dass nach Zugabe von Wasser und Nährmedium dann diese Keime ohne Verzögerungen in ihrer Gesamtheit zu wachsen beginnen, so dass sie für kontrollierte Wachstumsversuche, wie sie bei der mikrobiologischen Vitaminbestimmung erfolgen, verwendet werden können .

Der Vorteil des erfindungsgemäßen Verfahrens liegt darin, dass der Mikroorganismus für die Vitaminbestimmung nicht mehr neu angezüchtet werden muss, sondern in den Vertiefungen einer Mikrotiterplatte in definierter Zahl und Beschaffenheit vorliegt. Damit entfallen für das Untersuchungslabor alle Zucht- und Verdünnungsschritte. Es müssen nur exakte Mengen vitamindefizientes Nährmedium sowie Probe beziehungsweise Vitamin-Standardkonzentrationen in die einzelnen Vertiefungen der Mikrotiterplatte zugesetzt werden. Da die Keime in den Vertiefungen über die Mikrotiterplatten stets gleich sind, ist der Messbereich insgesamt weniger variant. Das heißt, die Mikrotiterplatten können für bestimmte Inkubationszeiten ausgelegt werden. Auch besteht eine viel geringere Gefahr von Verdünnungsfehlern, da letztlich nur die Probe in einfachen Schritten verdünnt werden muss. Die Lösungen mit festen Standardkonzentrationen des zu untersuchenden Vitamins können dem Teilesatz beigelegt werden. Damit werden die Standardkurven zuverlässiger. Die Kontaminationsgefahr ist reduziert. Die Wachstumsmedien sind charakterisiert und für den jeweiligen Keim vorgetestet. Mit der Vitaminbestimmung kann daher sofort begonnen werden, denn Keimzahl, Wachstumszustand, Genotyp des Keims und Testmedium sind überprüft, und für die Mikrotiterplatte, die Keime und die Testmedien kann eine Haltbarkeit von mindestens 12 Monaten garantiert werden. Die Platten und die Zutaten müssen nur trocken und keimfrei bei Raumtemperatur, ideal bei 4°C gelagert werden.

Durch die Mikrotiterplatte wird die Bestimmung ferner üblichen ELISA und RIA-Verfahren angepasst. Die Mikrotiterplattentechnik erlaubt größte Probenzahlen, den Einsatz von Pipettierautomaten und eine automatisierte Auslesung und Bestimmung von Keimwachstum und Stoffwechselumsatz.

Verfahrenstechnisch wird die Keimsuspension in einer spezifisch vitamindefizienten Lösung in Gegenwart von Trehalose schockgefroren und gefriergetrocknet. Auch in der Natur überleben Keime, indem sie sich vor dem völligen Austrocknen mit einer glasartigen Zuckerschicht schützen. Dieser natürliche Überlebensmechanismus wird erfindungsgemäß genutzt.

Im Gegensatz zur Gefrierlagerung sind die Zellen nach der erfindungsgemäßen Trehalose- Trockenlagerung sofort bei Zugabe von Flüssigkeit beziehungsweise Wasser wieder aktiv. Der übliche Gefrierschock der Zellen entfällt. Die Stabilität der Zellen wird gewährleistet durch den Zusatz von Trehalose zum Medium Einfrier- und Lagermedium. Die Trehalose und die Sucrose bilden mit den Proteinen der Zelle Wasserstoffbrückenbindungen, so dass die Zellen im natürlichen Trockenzustand über Monate haltbar sind. Es bietet sich an, den Zellen Alkali- und Erdalkaliionen anzubieten wie Magnesium, Calcium, Kalium, Natrium. Für jeden Stamm wird der Puffer für die Trockenlagerung und zur Rekonstituierung der Keime zu optimieren sein. In der Regel wurden bislang übliche Tris- und PBS- Lösungen mit je 10 mmol/L

$Mg^{2+}$, $Ca^{2+}$ und $Zn^{2+}$ als Lagerpuffer verwendet. Wenngleich sie verwendet werden können, bergen sie den Nachteil, dass gefördert durch den Stress der Gefriertrocknung und Trockenlagerung sich einige Keime bereits auf eine Mangelumgebung einstellen, mutieren oder anderweitig umorientieren, so dass bei Zusatz von Wasser beziehungsweise Probe Keime vorliegen, die ohne Vitamin wachsen können. Es ist daher vorteilhaft, den Mikroorganismus im Assaymedium (ohne Bestimmungssubstanz) in Gegenwart konservierender Trehalose bei -80°C schockzugefrieren und gefrierzutrocknen. So konservierte Bestimmungskeime sind über Monate bei Raumtemperatur stabil, verändern sich nicht und nach Zusatz von Assaymedium mit und ohne Probe wachsen nur die Keime, welche die Bestimmungssubstanz benötigen. Da durch dieses Vorgehen wirklich keine Keime vorliegen, die ohne das Vitamin beziehungsweise die Bestimmungssubstanz wachsen können, kann das Wachstum in einem Verbrauchsversuch (Runoff- Verfahren) bestimmt werden. Mit anderen Worten: 48 Stunden nach Zusatz von Wasser und vitaminhaltiger Probe haben sich dann beispielsweise die Keime genau so weit vermehrt, bis alle Bestimmungssubstanz verbraucht ist. Alle anderen für das Wachstum notwendige Substanzen sind im Überschuss vorhanden. Ist die Bestimmungssubstanz verbraucht, stellt der Keim das Wachstum ein. Da die Bestimmung von Endpunkten sehr viel weniger fehleranfällig und bequemer ist als die Bestimmung von relativen Wachstumsgeschwindigkeiten, wird hierdurch insgesamt die Bestimmung genauer und reproduzierbarer.

Der Vorteil der erfindungsgemäßen Trockenkeim-Mikrotiterplatte liegt darin, (i) dass bei einer Massenproduktion eine aufwendige Kalibrierung der Zellzahl pro Vertiefung erfolgen kann; (ii) dass die vorbereiteten Trockenkeim-Mikrotiterplatten vorab auf Funktionstüchtigkeit und optimale beziehungsweise Mindestinkubationszeiten getestet werden können - diese Werte stehen dem Anwender als Kenndaten der Trockenkeim-Mikrotiterplatte zur Verfügung - und (iii) dass eine Standard-Mikrotiterplatte mit 96 Vertiefungen gleichzeitig mehrere Mess- und Vergleichsreihen mit Proben und Vergleichsstandards erlaubt. Für das Labor entfallen somit nicht nur die Vorbereitungsschritte (Validierungsstudien, Überprüfung der Genauigkeit der Analysen, Charakterisierung von Standard und Testmedium), sondern alle Schritte können auch in automatischen Verfahren oder durch Analyseautomaten erfolgen.

Durch Zugabe von Wasser oder Nährlösung können die Impfkeime in den Vertiefungen der Mikrotiterplatte für sofortige Wachstumsversuche homogen revitalisiert werden. Damit können alle Reagenzien für eine mikrobiologische Vitaminbestimmung in einem Kit gebrauchsfertig in den Handel gebracht werden. Nicht nur, dass mit den Wachstumsversuchen dann sofort begonnen werden kann, sondern bei der manuellen Analyse stellt dies generell eine wesentliche Arbeitserleichterung dar bei Steigerung von Qualität und Genauigkeit. Die Mikrotiterplattentechnik erlaubt ferner eine Adaption auf Analysevoll- und -halbautomaten. Weitere Vorteile, Aufgaben und Ausführungsformen der Erfindung sind den nachstehenden Beispielen und den Abbildungen zu entnehmen.

## KURZE BESCHREIBUNG DER ABBILDUNGEN

Es zeigt:

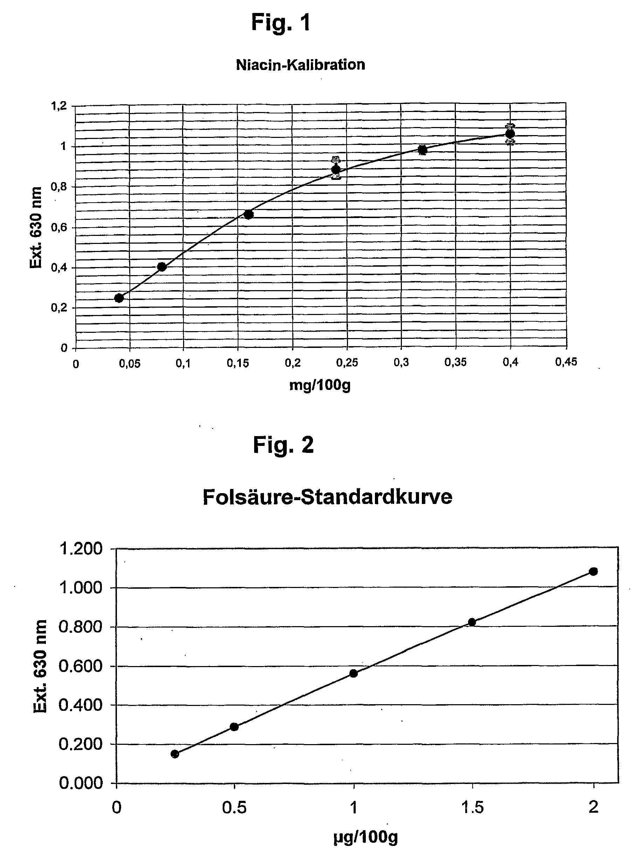

Figuren 1-7 graphische Darstellung des Wachstums von verschiedenen Mikroplatten-Trockenkeimen gemäß der Erfindung in Abhängigkeit der Konzentration von verschiedenen Vitaminen im jeweiligen Testmedium.

## EINGEHENDE BESCHREIBUNG BEVORZUGTER AUSFÜHRUNGSFORMEN

BEISPIELE

*Beispiel 1 - Niacin-Bestimmung (Vitamin-B3)*

*1. Vorbereitung der Mikrotiterplatte*

Es wurde eine Kultur von *Lactobacillus plantarum* (ATCC 8014) aus einem Glycerol-Stock in 10 ml Lactobacillus-Medium übergeimpft und 36 Stunden bebrütet. Die Kultur wurde in der logarithmischen Phase durch Abzentrifugieren (2500 G x 5 Minuten) gestoppt, das resultierende Zellpellet 3 x in 0,85% NaCl-Lösung gewaschen, in 10 ml 200 mmol/L Trehalose, 10 mmol/L $CaCl_2$ suspendiert, und dann in etwa 1 zu 10 verdünnt mit Difco™ Niacin-Assaymedium, enthaltend Casaminosäuren 12,0 g/L, Dextrose 40,0 g/L, Natriumacetat 20,0 g/L, L-Cystin 0,4 g/L, DL-Tryptophan 0,2 g/L, Adeninsulfate 20,0 mg/L, Guanine-Hydrochlorid 20,0 mg/L, Uracil 20,0 mg/L, Thiamin-Hydrochlorid 200,0 μg/L, Calcium-Pantothenat 200,0 μg/L, Pyridoxin-Hydrochlorid 400,0 μg/L, Riboflavin 400,0 μg/L, p-Aminobenzoesäure 200,0 μg/L, Biotin 0,8 μg/L, Dikaliumhydrogenphosphat 1,0 g/L, Kaliumdihydrogenphosphat 1,0 g/L, Magnesiumsulfat 0,4 g/L, Natriumchlorid 20,0 mg/L, Eisensulfat 20,0 mg/L, Mangansulfat 20,0 mg/L sowie 200 mmol/L Trehalose, 10 mmol/L $CaCl_2$. Die Verdünnung wurde so eingestellt, dass 1 ml Keimsuspension $10^7$ lebensfähige Keime enthielt. Es wurde 3 μl Keimsuspension in jede Kavität der Mikrotiterplatte gegeben, die Keimsuspension in der Kavität bei -80°C im Gefrierschrank schockgefroren und das Keimpellet am Boden der Kavität durch Anlegen von Vakuum gefriergetrocknet. Jede Vertiefung der Mikrotiterplatte enthielt so genau $3x10^4$ lebensfähige Keime *Lactobacillus* plantarum im gleichen Wachstumsstadium und zwar verpackt in einem winzigen Trehalose-/Zucker-/Salzpellet. Es ist darauf zu achten, dass ein klebriges Pellet erzielt wird, da ansonsten das Pellet beim Transport aus der Kavität fallen kann. Ansonsten ist ein klebriger Zucker wie Saccharose oder Dextrose der Einfrierlösung zuzusetzen. Die Platten wurden steril und mit Trockenmittel (Sica) lichtdicht verpackt. So konfektionierte Mikrotiterplatten waren über längere Zeiträume bei Raumtemperatur haltbar, ohne dass dies auf die Wachstumsfähigkeit der Keime einen Einfluss hatte.

*2. Mikrobiologische Bestimmung*

Es wurde Niacin (Nikotinsäure und Nikotinsäureamid) quantitativ in Lebensmitteln bestimmt. Das Verfahren ist nicht anwendbar bei Proben, die antibiotische oder wachstumshemmende Stoffe enthalten. Niacytin wird von *Lactobacillus plantarum* (ATCC 8014) nicht verwertet. Die Niacin-Konzentration wurde über die durch Bakterienwachstum erfolgten Trübung im Vergleich zu einer Standardreihe gemessen.

*Material*: Alle Glasgefäße und Gerätschaften für die Verdünnungsreihe wurden von Hand mit einer ca. 1%igen Tween® 80- Lösung vorgewaschen, dann mit Salzsäure (0,1 M) und mit NaOH-Lösung (0,1 M) behandelt, dreimal mit Leitungswasser heiß und kalt gespült und schließlich mit destilliertem Wasser gewaschen. Die Gefäße und Geräte wurden getrocknet, mit geeigneten Verschlüssen (Metallkappen, Aluminiumfolie) versehen und umwickelt und länger als eine Stunde bei mindestens 250°C behandelt.

*Probenlagerung:* Säfte und andere verderbliche Proben wurden bis zum Zeitpunkt der Untersuchung gekühlt gelagert.

*Vitaminstandard:* 10 mg Niacin wurde mit einer Präzisionswaage in ein geeignetes Glasgefäß eingewogen und unter Rühren in 100 ml Wasser gelöst. Die Konzentration der so hergestellten Stammlösung betrug 0,1 mg Niacin pro Milliliter (= 100000 ng/ml). Diese Lösung wurde in zwei Schritten zunächst 1:100 und dann 1:25 auf eine Endkonzentration von 40 ng Niacin/ml verdünnt. Weitere Verdünnungsstufen wurden angefertigt (0,6 ng/150$\mu$l, 1,2 ng/150$\mu$l, 2,4 ng/150$\mu$l, 3,6 ng/150$\mu$l, 4,8 ng/150$\mu$l, 6,0 ng/150$\mu$l = Standardreihe)

*Probenaufarbeitung.* Zwischen 1 und 10 g Probe wurden in einem Glasgefäß mit Rührstift eingewogen und mit Wasser auf 100 g aufgefüllt. Das Gefäß wurde verschlossen und bei 120°C 2 min sterilisiert. Die Probe wurde dann so verdünnt, dass die zu erwartende Niacin-Konzentration bei ca. 1,2 ng/150$\mu$l lag. Die Proben wurden hierzu im Doppelansatz mit einer Reihe unterschiedlicher Probenverdünnungen extrahiert.

*Bebrütung:* In die Vertiefungen einer Mikrotiterplatte wurde 150 $\mu$l Standard- und Probenlösung sowie 150$\mu$l Niacin- defizientes doppelkonzentriertes Assaymedium (Niacin- Medium) zugegeben und mit einer Luft und Wasser undurchlässigen Folie verschlossen. Die Bebrütung erfolgte für 48 h bei einer Temperatur von 37 °C im Brutschrank.. Die gewählte Temperatur wurde in einem Bereich von $\pm$ 0, 5°C konstant gehalten. Zwischen Mediumzusatz und dem Beginn der Bebrütung lagen nicht mehr als 30 Minuten.

*Auswertung:* Nach Ende der Bebrütung wurde die Trübung der Standards und Proben auf der Mikrotiterplatte mit einem ELISA- Reader (ELISA- Photometer) bei 630 nm (alternativ 540 nm) gemessen. Aus den Werten des Ansatzes wurde der Mittelwert für die jeweilige Konzentrationsstufe berechnet.

*Aufstellen der Kalibrierkurve:* Die Extinktionswerte der Standardreihe wurde auf Millimeterpapier aufgetragen bzw. mittels geeigneter Software graphisch dargestellt. Die Funktion der den ermittelten Datenpunkten zugehörigen polynomischen Trendlinie 4. Ordnung wurde errechnet. Das Quadrat des Korrelations-Koeffizienten der Werte muss mindestens mehr als 0,990 betragen. Der Korrelationskoeffizient errechnet sich dabei wie folgt:

$$r = \frac{n\left(\sum xy\right) - \left(\sum x\right)\left(\sum y\right)}{\sqrt{\left[n\sum x^2 - \left(\sum x\right)^2\right]\left[n\sum y^2 - \left(\sum y\right)^2\right]}}$$

*Berechnung:* Durch Einsetzen der ermittelten Proben-Extinktionswerte in y der Funktionsgleichung und mittels der pq-Formel kann der zugehörige x-Wert errechnet werden. Hilfsweise kann der gesuchte x-Wert aus der graphisch erstellen Eichkurve abgelesen werden. Bei Probenverdünnungen mit starker Eigentrübung wurden die ermittelten y-Werte noch im Vergleich zum Messwert einer mitgeführten Sterilkontrolle korrigiert. Der Korrekturwert ergibt sich aus der Messdifferenz zwischen mitgeführter Sterilkontrolle und einer nicht beimpften (getrübten) Probe nach Inkubation im Wasserbad.

Es empfiehlt sich bei jeder fünften Untersuchung als Maßnahme zur Qualitätssicherung eine definierte Probe mitzuführen. Die Ergebnisse sind in einer Mittelwert-Regelkarte einzutragen; die Karte ist mit den aktuellen Rohdaten abzulegen.

Figur 1 zeigt die ermittelte Standardkurve nach 48 Stunden Inkubation von 5x10$^4$ Trockenzellen in Niacin-Assaymedium. Trotz der Gefriertrocknung in der Mikrotiterplatte und 4wöchiger Lagerung der Trockenkeim-Mikrotiterplatte bei Raumtemperatur wurden stetige Wachstumskurven mit *Lactobacillus plantarum* erhalten, welche die Bestimmung von Vitamin-B$_3$ in Standard und Proben erlaubten.

In einem weiteren Vergleichsversuch wurden gleiche Zellzahlen *Lactobacillus plantarum* 72 Stunden in PBS, 20% Glycerin eingefroren. Im nachfolgenden Wachstumsversuch waren die Glycerin-stabilisierten Zellen zwar lebensfähig, das festgestellte Wachstum korrelierte in keiner Weise mit der Menge an Niacin im Assaymedium. Vielmehr war über alle Niacin-Konzentrationen ein nahezu gleiches Wachstum festzustellen. Hier stammte also das Wachstum in allen Fällen von wenigen Startzellen, also von wenigen Zellen, die in der Lösung einen Startvorsprung hatten.

Anders bei den erfindungsgemäß, in Gegenwart eines konservierenden Zuckers getrockneten Keimen von *Lactobacillus plantarum.* Hier tragen, wie die konzentrationsabhängige Wachstumskurve zeigt, alle eingesetzten Zellen am Umsatz und Wachstum in der zugegebenen Standard- und Probenlösung bei. Die Wachstumskurve ist dann nur noch abhängig vom vorhandenen Niacin.

TABELLE 1

| Bestimmung der Niacin-Standardkurve und 8 Proben | | | | | |
|---|---|---|---|---|---|
| **Standard mg/100g** | **Messung bei 630 nm** | **Anzahl** | **Mittelwert** | **Standardabweichung** | **Varianz (%)** |
| 0,04 | 0,242 0,247 0,246 | 3 | 0,245 | 0,003 | 1,08 |
| 0,08 | 0,405 0,399 0.398 | 3 | 0,401 | 0,004 | 0,945 |
| 0,16 | 0,653 0,654 0,663 | 3 | 0,657 | 0,006 | 0,839 |
| 0,24 | 0,849 0,926 0,854 | 3 | 0,876 | 0,043 | 4,917 |
| 0,32 | 0,961 0,972 0,979 | 3 | 0,971 | 0,009 | 0,935 |
| 0,4 | 1,055 1,085 1,008 | 3 | 1,049 | 0,039 | 3,699 |
| Probe SPL1 | 0,416 0,413 0,415 | 3 | 0,415 | 0,002 | 0,368 |
| SPL2 | 0,551 0,562 0,556 | 3 | 0,556 | 0,006 | 0,99 |
| SPL3 | 0,898 0,892 0,906 | 3 | 0,899 | 0,007 | 0,782 |
| SPL4 | 1,077 1,102 1,096 | 3 | 1,092 | 0,013 | 1,196 |
| SPL5 | 0,299 0,298 0.295 | 3 | 0,297 | 0,002 | 0,7 |
| SPL6 | 0,394 0,409 0,396 | 3 | 0,4 | 0,008 | 2,038 |

(fortgesetzt)

| Bestimmung der Niacin-Standardkurve und 8 Proben | | | | | |
|---|---|---|---|---|---|
| Standard mg/100g | Messung bei 630 nm | Anzahl | Mittelwert | Standardabweichung | Varianz (%) |
| SPL7 | 0,672<br>0,674<br>0,68 | 3 | 0,675 | 0,004 | 0,616 |
| SPL8 | 0,866<br>0,866<br>0.866 | 3 | 0,866 | 0 | 0 |

Die Konzentrationsangabe richtet sich nach der üblichen Gehaltsangabe in der Lebensmittelchemie. Hierbei werden die Vitamine von 1 g Probeeinwaage in 100 ml Wasser extrahiert, 150 $\mu$l Extrakt in eine Kavität der Mikrotiterplatte überführt, mehrfach konzentriertes Testmedium zugesetzt, die Menge an Vitamin bestimmt und schließlich auf 100 g Probe hochgerechnet. Der Standard wurde dementsprechend mit angesetzt.

*Beispiel 2 - Folsäure-Bestimmung*

Die Vorbereitung der Mikrotiterplatte und die Bestimmung von Folsäure (Pteroylglutaminsäure und sonstige Folsäurekonjugate) erfolgten wie in Beispiel 1, nur dass als Testorganismus *Lactobacillus rhamnosus ATCC* 7469 verwendet wurde. Als Assaymedium wurde Difco™ Folsäure-Casein-Medium verwendet, enthaltend Aktivkohle behandeltes pankreaseverdautes Casein 10,0 g/L, Dextrose 40,0 g/L, Natriumacetat 40,0 g/L, Kaliumdihydrogenphosphat 1,0 g/L, Dikaliumhydrogenphosphat 1,0 g/L, DL-Tryptophan 0,2 g/L, L-Asparagin 0,6 g/L, L-Cystein-Hydrochlorid 0,5 g/L, Adeninsulfat 10,0 mg/L, Guanin-Hydrochlorid 10,0 mg/L, Uracil 10,0 mg/L, Xanthin 20,0 mg/L, Polysorbat-80 0,1 g/L, Glutathion (reduziert) 5,0 mg/L, Magnesiumsulfat 0,2 g/L, Natriumchlorid 20,0 mg/L, Eisensulfat 20,0 mg/L, Mangansulfat 15,0 mg/L, Riboflavin 1,0 mg/L, p-Aminobenzoesäure 2,0 mg/L, Pyridoxin-Hydrochlorid 4,0 mg/L, Thiamin-Hydrochlorid 400,0 $\mu$g/L, Calciumpantothenat 800,0 $\mu$g/L, Nikotinsäure 800,0 $\mu$g/L, Biotin 20,0 $\mu$g/L, 0,05% Ascorbinsäure. Das Lagermedium enthielt 200 mmol/L Trehalose. Der Folsäurestandard war in 100 mmol/L Kaliumphosphatpuffer, pH 6.1, 0,1 % Ascorbinsäure gelöst.

Tabelle 2

| Folsäure-Standardkurve | | | | | | |
|---|---|---|---|---|---|---|
| Standard pg/150$\mu$l | Standard $\mu$g/100g | Messung bei 630 nm | Anzahl | Mittelwert | Standardabweichung | Varianz (%) |
| 3,75 | 0,25 | 0,261<br>0,261<br>0,269 | 3 | 0,264 | 0,005 | 1,752 |
| 7,5 | 0,5 | 0,556<br>0,536<br>0,544 | 3 | 0,545 | 0,01 | 1,846 |
| 15 | 1 | 0,784<br>0,788<br>0,807 | 3 | 0,793 | 0,012 | 1,55 |
| 22,5 | 1,5 | 0,986<br>1,002<br>1,01 | 3 | 0,999 | 0,012 | 1,223 |
| 30 | 2 | 1,121<br>1,074<br>1,087 | 3 | 1,094 | 0,024 | 2,218 |

Die ermittelte Standardkurve ist in Figur 2 graphisch dargestellt.

*Beispiel 3 - Vitamin B$_{12}$-Bestimmung*

Die Vorbereitung der Mikrotiterplatte und die Bestimmung von Vitamin B12 (Cyanocobalamin, Hydroxycobalamin) erfolgten wie in Beispiel 1, nur dass als Testorganismus *Lactobacillus delbrueckii subsp. lactis* (L. leichmanii) ATCC 7830 verwendet wurde. Als Assaymedium diente Difco™ B$_{12}$- Assaymedium, enthaltend Casaminosäuren 15, 0 g/L, Dextrose 40, 0 g/L, Asparagin 0, 2 g/L, Natriumacetat 20, 0 g/L, Ascorbinsäure 4, 0 g/L, L- Cystein 4, 0 g/L, DL-Tryptophan 0, 4 g/L, Adeninsulfat 20, 0 mg/L, Guanin- Hydrochlorid 20, 0 mg/L, Uracil 20, 0 mg/L, Xanthin 20, 0 mg/L, Polysorbat- 80 2, 0 g/L, Magnesiumsulfat (wasserfrei) 0, 4 mg/L, Natriumchlorid 20, 0 mg/L, Eisensulfat 20, 0 mg/L, Mangansulfat 20, 0 mg/L, Riboflavin 1, 0 mg/L, p- Aminobenzoesäure 2, 0 mg/L, Pyridoxin- Hydrochlorid 4, 0 mg/L, Thiamin- Hydrochlorid 1, 0 mg/L, Calciumpantothenat 1, 0 mg/L, Niacin 2, 0 mg/L, Biotin 10, 0 $\mu$g/L, . Pyridoxin- Hydro-chlorid 4, 0 mg/L, Pyridoxal- Hydrochlorid 4, 0 mg/L, Pyridoxamin- Hydrochlorid 800, 0 $\mu$g/L, Folsäure 200, 0 $\mu$g/L, Kaliumdihydrogenphosphat 1, 0 g/L, Dikaliumhydrogenphosphat 1, 0 g/L. Das Lagermedium enthielt zudem 200 mmol/L Trehalose.

TABELLE 3

| Vitamin-B$_{12}$-Standardkurve | | | | | |
|---|---|---|---|---|---|
| Standard $\mu$g/100g | Messung bei 630 nm | Anzahl | Mittelwert | Standardabweichung | Varianz (%) |
| 0,1 | 0,264 0,271 0,269 | 3 | 0,268 | 0,004 | 1,345 |
| 0,2 | 0,531 0,51 0,503 | 3 | 0,515 | 0,015 | 2,831 |
| 0,3 | 0,727 0,721 0,736 | 3 | 0,728 | 0,008 | 1,037 |
| 0,4 | 0,88 0,863 0,865 | 3 | 0,869 | 0,009 | 1,069 |
| 0,8 | 1,086 1,073 1,048 | 3 | 1,069 | 0,019 | 1,807 |

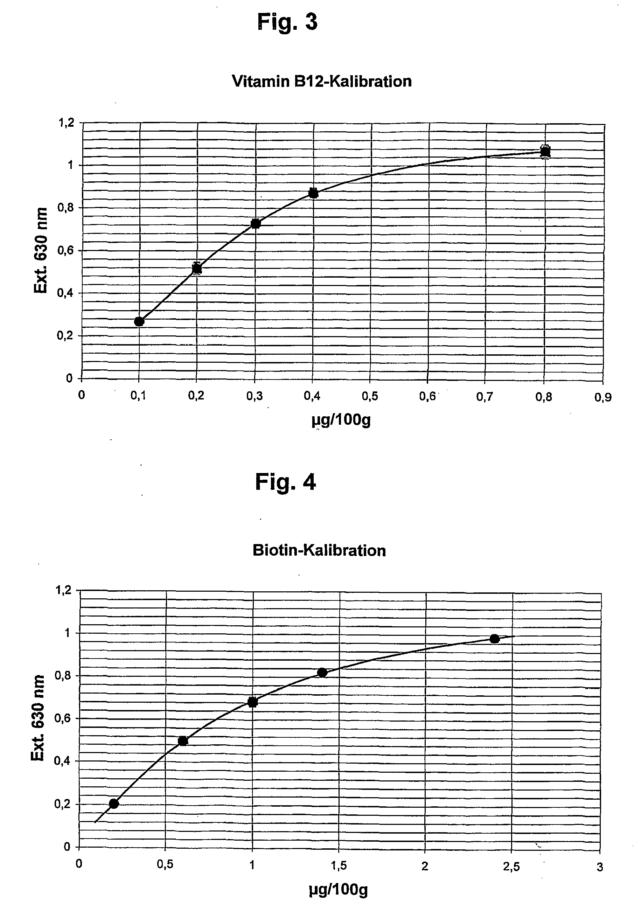

Die ermittelte Vitamin- B$_{12}$- Standardkurve ist in Figur 3 graphisch dargestellt.

*Beispiel 4 - Biotin-Bestimmung*

Die Vorbereitung der Mikrotiterplatte und die Bestimmung von Biotin erfolgten wie in Beispiel 1 mit *Lactobacillus plantarum* ATCC 8014. Als Assaymedium diente Difco™ Biotin-Assaymedium, enthaltend Casaminosäuren 12,0 g/L, Dextrose 40,0 g/L, Natriumacetat 20,0 g/L, L-Cystein 0,2 g/L, DL-Tryptophan 0,2 g/L, Adeninsulfat 20,0 mg/L, Guanin-Hydrochlorid 20,0 mg/L, Uracil 20,0 mg/L, Thiamin-Hydrochlorid 2,0 mg/L, Riboflavin 2,0 mg/L, Niacin 2,0 mg/L, Calciumpantothenat 2,0 mg/L, Pyridoxin-Hydrochlorid 4,0 mg/L, p-Aminobenzoesäure 0,2 mg/L, Magnesiumsulfat (wasserfrei) 0,4 mg/L, Natriumchlorid 20,0 mg/L, Eisensulfat 20,0 mg/L, Mangansulfat 20,0 mg/L, , Kaliumdihydrogenphosphat 1,0 g/L, Dikaliumhydrogenphosphat 1,0 g/L. Das Lagermedium enthielt zudem 200 mmol/L Trehalose. Die ermittelte Standardkurve ist in Figur 4 gezeigt.

TABELLE 4

| Biotin-Standardkurve | | | | | |
|---|---|---|---|---|---|
| Standard $\mu$g/100 g | Messung bei 630 nm | Anzahl | Mittelwert | Standardabweichun g | Varianz (%) |
| STD1 | 0,205 | 3 | 0,204 | 0,001 | 0,49 |
| | 0,204 | | | | |

(fortgesetzt)

| Biotin-Standardkurve | | | | | |
|---|---|---|---|---|---|
| Standard μg/100 g | Messung bei 630 nm | Anzahl | Mittelwert | Standardabweichun g | Varianz (%) |
| | 0,203 | | | | |
| STD2 | 0,491 0,506 0,5 | 3 | 0,499 | 0,008 | 1,513 |
| STD3 | 0,676 0,693 0,686 | 3 | 0,685 | 0,009 | 1,247 |
| STD4 | 0,826 0,822 0,82 | 3 | 0,823 | 0,003 | 0,371 |
| STD5 | 0,986 0,988 0,982 | 3 | 0,985 | 0,003 | 0,31 |

Die zugehörige Standardkurve ist in Figur 4 gezeigt.

*Beispiel 5- B$_6$-Pyridoxin-Bestimmung*

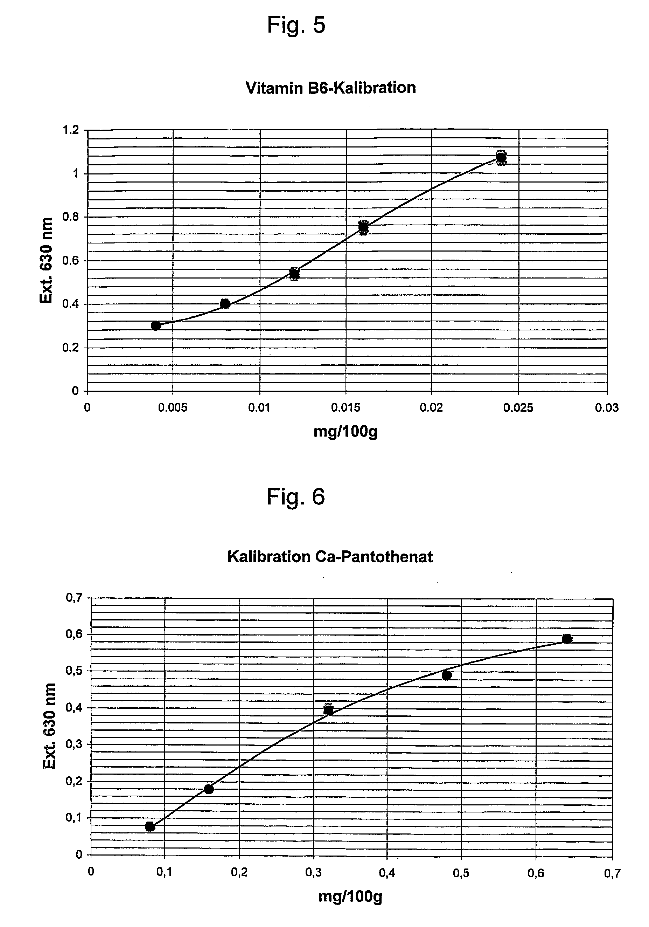

Die Vorbereitung der Mikrotiterplatte und die Bestimmung von B6-Pyridoxin erfolgten wie in Beispiel 1, nur dass als Testorganismus *Saccharomyces cerevisae* ATCC 9080 verwendet wurde. Als Assaymedium wurde Difco™ Pyridoxine-Y-Medium verwendet, enthaltend Dextrose 40,0 g/L, L-Asparagin 4,0 g/L, Ammoniumsulfat 4,0 g/L , Kaliumdihydrogenphosphat 3,0 g/L, Magnesiumsulfat 1,0 g/L, Calciumchlorid 0,49 g/L, DL-Methionin 40,0 mg/L, DL-Tryptophan 40,0 mg/L, DL-Isoleucin 40,0 mg/L, DL-Valin 40,0 mg/L, L-Histidin-Hydrochlorid 20,0 mg/L, Riboflavin 20,0 mg/L, Biotin 8,0 mg/L, Inositol 5,0 mg/L, Eisensulfat 500 μg/L, Thiamin-Hydrochlorid 400,0 μg/L, Calciumpantothenat 400,0 μg/L, Nikotinsäure 400,0 μg/L, Borsäure 200,0 μg/L, Kaliumjodid 200,0 μg/L, Ammoniummolybdat 40,0 μg/L, Mangansulfat 80,0 μg/L, Kupfersulfat 90,0 μg/L, Zinksulfate 80,0 μg/L Das Lagermedium enthielt zudem 200 mmol/L Trehalose. Die ermittelte Standardkurve ist in Figur 5 gezeigt.

TABELLE 5

| B$_6$-Pyridoxin-Standardkurve | | | | | |
|---|---|---|---|---|---|
| Standard mg/100 g | Messung bei 630 nm | Anzahl | Mittelwert | Standardabweichung | Varianz (%) |
| 0,004 | 0,298 0,297 0,301 | 3 | 0,299 | 0,002 | 0,697 |
| 0,008 | 0,394 0,407 0,398 | 3 | 0,4 | 0,007 | 1,666 |
| 0,012 | 0,522 0,541 0,552 | 3 | 0,538 | 0,015 | 2,819 |
| 0,016 | 0,73 0,766 0,762 | 3 | 0,753 | 0,02 | 2,622 |
| 0,024 | 1,049 1,088 1,07 | 3 | 1,069 | 0,02 | 1,826 |

*Beispiel 6 - Calciumpantothenat-Bestimmung*

Die Vorbereitung der Mikrotiterplatte und die Bestimmung von Calciumpantothenat erfolgten wie in Beispiel 1 mit *Lactobacillus plantarum* ATCC 8014. Als Assaymedium wurde Difco™ Pantothenat-Assaymedium verwendet, enthaltend Casaminosäuren 10,0 g/L, Dextrose 40,0 g/L, Natriumacetat 20,0 g/L, L-Cystine 0,4 g/L, DL-Tryptophan 0,2 g/L, Adeninsulfat 20,0 mg/L, Guanin-Hydrochlorid 20,0 mg/L, Uracil 20,0 mg/L, Thiamin-Hydrochlorid 200 $\mu$g/L, Riboflavin 200 $\mu$g/L, Niacin 1,0 mg/L, Pyridoxin 800 $\mu$g/L, p-Aminobenzoesäure 200,0 $\mu$g/L, Biotin 1,0 $\mu$g/L, Kaliumdihydrogenphosphat 1,0 g/L, Dikaliumhydrogenphosphat 1,0 g/L, Magnesiumsulfat 0,4 g/L, Natriumchlorid 20,0 mg/L, Eisensulfat 20,0 mg/L, Mangansulfat 20,0 mg/L Das Lagermedium enthielt zudem 200 mmol/L Trehalose. Die ermittelte Standardkurve ist in Figur 6 gezeigt.

TABELLE 6

| Ca Pantothenat-Standardkurve | | | | | |
|---|---|---|---|---|---|
| Standard mg/100 g | Messung bei 630 nm | Anzahl | Mittelwert | Standardabweichung | Varianz (%) |
| 0,08 | 0,075 0,08 0,073 | 3 | 0,076 | 0,004 | 4,744 |
| 0,16 | 0,181 0,179 0,178 | 3 | 0,179 | 0,002 | 0,852 |
| 0,32 | 0,389 0,404 0,392 | 3 | 0,395 | 0,008 | 2,009 |
| 0,48 | 0,49 0,493 0,491 | 3 | 0,491 | 0,002 | 0,311 |
| 0,64 | 0,589 0,594 0,588 | 3 | 0,59 | 0,003 | 0,545 |

*Beispiel 7 - Riboflavin-Bestimmung*

Die Vorbereitung der Mikrotiterplatte und die Bestimmung von Riboflavin erfolgten wie in Beispiel 1, nur dass *Lactobacillus rhamnosus* ATCC 7469 als Testorganismus verwendet wurde. Als Assaymedium wurde Difco™ Riboflavin-Assaymedium verwendet, enthaltend Cas aminosäuren 10,0 g/L, Dextrose 20,0 g/L, Natriumacetat 15,0 g/L, Dikaliumhydrogenphosphat 1,0 g/L, Kaliumdihydrogenphosphat 1,0 g/L, L-Asparagin 0,6 g/L, L-Cystine 0,2 g/L, DL-Tryptophan 0,2 g/L, Magnesiumsulfat 0,4 g/L, Adeninsulfat 20,0 mg/L, Guanin-Hydrochlorid 20,0 mg/L, Uracil 20,0 mg/L, Xanthin 20 mg/L, Eisensulfat 20,0 mg/L, Mangansulfat 20,0 mg/L, , Natriumchlorid 20,0 mg/L, Pyridoxin-Hydrochlorid 4,0 mg/L, Pyridoxal-Hydrochlorid 4,0 mg/L, p-Aminobenzoesäure 2,0 mg/L, Calciumpantothenat 800 $\mu$g/L, Folsäure 800 $\mu$g/L, Nikotinsäure 800 $\mu$g/L, Thiamin-Hydrochlorid 400 $\mu$g/L, Biotin 1,0 $\mu$g/L. Das Lagermedium enthielt zudem 200 mmol/L Trehalose.

TABELLE 7

| $B_2$ Riboflavin - Standardkurve | | | | | |
|---|---|---|---|---|---|
| Standard mg/100g | Messung bei 630 nm | Anzahl | Mittelwert | Standardabweichung | Varianz (%) |
| 0,1 | 0,196 0,209 0,206 | 3 | 0,204 | 0,007 | 3,342 |
| 0,2 | 0,383 0,404 0,408 | 3 | 0,398 | 0,013 | 3,371 |

# EP 1 774 021 B1

(fortgesetzt)

| B₂ Riboflavin - Standardkurve | | | | | |
| --- | --- | --- | --- | --- | --- |
| Standard mg/100g | Messung bei 630 nm | Anzahl | Mittelwert | Standardabweichung | Varianz (%) |
| 0,3 | 0,624 0,616 0,628 | 3 | 0,623 | 0,006 | 0,981 |
| 0,4 | 0,752 0,755 0,771 | 3 | 0,759 | 0,01 | 1,345 |
| 0,5 | 0,855 0,886 0,9 | 3 | 0,88 | 0,023 | 2,616 |

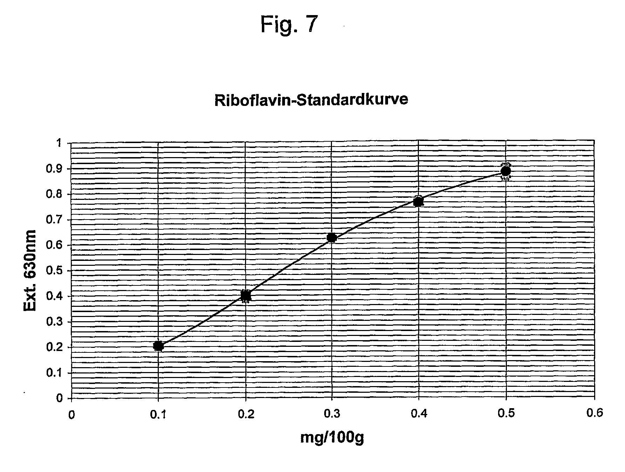

Die ermittelte Standardkurve ist in Figur 7 gezeigt

Der Schutzbereich der Erfindung ergibt sich aus den nachstehenden Ansprüchen, welche gleichfalls zur Offenbarung gehören.

**Patentansprüche**

1. Verfahren zur quantitativen mikrobiologischen Bestimmung von einzelnen Vitaminen, Aminosäuren oder anderen lebensnotwendigen Stoffen in einem Stoffgemisch, **dadurch gekennzeichnet, dass** in dem Kulturgefäß für die mikrobiologische Wachstums- und Stoffwechselbestimmung
eine vorbestimmte Zahl vitaler Zellen eines geeigneten Mikroorganismus in einer Einfrierlösung in Gegenwart von 200 bis 500 mmol/L Trehalose schockgefroren und gefriergetrocknet werden, und dass
die Zellen in dem Kulturgefäß bis zur Verwendung trocken gelagert werden.

2. Verfahren nach Anspruch 1, wobei die Einfrierlösung 200 bis 250 mmol/L Trehalose enthält.

3. Verfahren nach Anspruch 1 oder 2, wobei die Einfrierlösung das Testmedium für die mikrobiologische Bestimmung ist.

4. Verfahren nach irgendeinem vorhergehenden Anspruch, wobei die Zellen bei einer Temperatur zwischen -10 und -100°C, bevorzugt zwischen -18 und -80°C schockgefroren und dann gefriergetrocknet werden.

5. Verfahren nach irgendeinem vorhergehenden Anspruch, wobei die Bestimmungssubstanz ausgewählt ist aus Vitamin- B₃ (Niacin, Nicotinsäure, Nicotinsäureamid), Vitamin B₁₂ (Cyanocobalamain, Hydroxycobalamin), Folsäure (Folsäure, Pteroylglutaminsäure, Folsäurekonjugate), Biotin, Pantothensäure (Panonthensäure, Panhenol), Vitamin B₁ (Thiamin, Thiaminpyrophosphat), Vitamin B₂ (Riboflavin, Riboflavinphosphat), Vitamin B₆ (Pyridoxin, Pyridoxal, Pyridoxamin, Pyridoxal- 5'- phosphat), Lysin, Methionin, Cystin, Cholin, Inositol.

6. Verfahren nach irgendeinem vorhergehenden Anspruch, wobei der Mikroorganismus ausgewählt ist aus *Lactobacillus delbrueckii subsp. Lactis, Enterococcus hirae ; Lactobacillus plantarum, Lactobacillus fermenti, Lactobacillus helveticus, L. veridescenc, Streptococcus faecalis.*

7. Verfahren nach irgendeinem vorhergehenden Anspruch, wobei das Kulturgefäß eine Mikrotiterplatte ist.

8. Testbesteck zur quantitativen mikrobiologischen Bestimmung von einzelnen Vitaminen, Aminosäuren oder anderen lebensnotwendigen Stoffen in einem Stoffgemisch, umfassend Kulturgefäße für die mikrobiologische Wachstums- und Stoffwechselbestimmung, wobei die Kulturgefäße die Vertiefungen einer Mikrotiterplatte sind die jeweils eine vorbestimmte Zahl vitaler Zellen eines geeigneten Mikroorganismus enthalten, welche durch Zusatz einer Einfrierlösung 200 bis 500 mmol/L Trehalose enthalten und durch Schockgefrieren und Gefriertrocknung für eine trockene Lagerung bei Umgebungstemperatur haltbar gemacht sind.

**9.** Testbesteck nach Anspruch 8, wobei der Mikroorganismus ausgewählt ist aus *Lactobacillus delbrueckii subsp. Lactis, Enterococcus hirae ; Lactobacillus plantarum, Lactobacillus fermenti, Lactobacillus helveticus, L. veridescenc, Streptococcus faecalis.*

## Claims

**1.** Method for the quantitative microbiological determination of individual vitamins, amino acids or other materials essential for life in a materials mixture, **characterised in that**, in the culturing vessel for the microbiological growth and metabolism determination,
a pre-determined number of vital cells of a suitable microorganism are shock-frozen and freeze-dried in a freezing solution in the presence of 200 to 500 mmol/L trehalose, and **in that**
the cells in the culturing vessel are stored in the dry until use.

**2.** Method according to claim 1, wherein the freezing solution comprises 200 to 250 mmol/L trehalose.

**3.** Method according to claim 1 or 2, wherein the freezing solution is the test medium for the microbiological determination.

**4.** Method according to any previous claim, wherein the cells are shock-frozen and freeze-dried at a temperature between -10 and -100°C, preferably between -18 and -80°C.

**5.** Method according to any previous claim, wherein the determination substance is selected from vitamin $B_3$ (niacin, nicotinic acid, nicotinic acid amide), vitamin $B_{12}$ (cyanocobalamine, hydroxycobalamine), folic acid (folic acid, pteroylglutamic acid, folic acid conjugate), biotin, pantothenic acid (pantothenic acid, panthenol), vitamin $B_1$ (thiamine, thiamine pyrophosphate), vitamin $B_2$ (riboflavine, riboflavine phosphate), vitamin $B_6$ (pyridoxine, pyridoxal, pyridoxamine, pyridoxal 5'- phosphate), lysine, methionine, cystine, choline, inositol.

**6.** Method according to any previous claim, wherein the microorganism is selected from *Lactobacillus delbrueckii subsp. Lactis, Enterococcus hirae; Lactobacillus plantarum, Lactobacillus fermenti, Lactobacillus helveticus, L. veridescenc, Streptococcus faecalis.*

**7.** Method according to any previous claim, wherein the culturing vessel is a microtiter plate.

**8.** Test kit for the quantitative microbiological determination of individual vitamins, amino acids or other materials essential for life in a materials mixture, comprising culturing vessels for the microbiological growth and metabolism determination, wherein the culturing vessels are the cavities of a microtiter plate, which each contain a predetermined number of vital cells of a suitable microorganism, which by the addition of a freezing solution contain from 200 to 500 mmol/L trehalose and are rendered durable for dry storage at ambient temperature by shock-freezing and freeze-drying.

**9.** Test kit according to claim 8, wherein the microorganism is selected from La*ctobacillus delbrueckii subsp. Lactis, Enterococcus hirae; Lactobacillus plantarum, Lactobacillus fermenti, Lactobacillus helveticus, L. veridescenc, Streptococcus faecalis.*

## Revendications

**1.** Procédé pour la détermination quantitative microbiologique de vitamines individuelles, d'acides aminés et d'autres composés essentiels à la vie dans un mélange de composés, **caractérisé en ce que**, dans le vaisseau de culture pour la détermination microbiologique de croissance et de métabolisme,
un nombre prédéterminé de cellules vitales d'un microorganisme approprié sont gelées en choc et lyophilisées en la présence de 200 à 500 mmol/L tréhalose, et **en ce que**
les cellules dans le vaisseau de culture sont stockées sèches jusqu'à leur utilisation.

**2.** Procédé selon la revendication 1, dans lequel la solution de gel comprend entre 200 et 250 mmol/L tréhalose.

**3.** Procédé selon la revendication 1 ou 2, dans lequel la solution de gel est le médium test pour la détermination microbiologique.

**4.** Procédé selon l'une quelconque revendication précédente, dans lequel les cellules sont gelées en choc et lyophilisées à une température entre -10 et -100°C, de préférence entre -18 et -80°C.

**5.** Procédé selon l'une quelconque revendication précédente, dans lequel la substance de détermination est sélectionnée parmi la vitamine $B_3$ (niacine, acide nicotinique, amide d'acide nicotinique), la vitamine $B_{12}$ (cyanocobalamine, hydroxycobalamine), l'acide folique (acide folique, acide ptéroylglutamique, conjugués de l'acide folique), la biotine, l'acide pantothénique (acide pantothénique, panthénol), la vitamine $B_1$ (thiamine, pyrophosphate de thiamine) la vitamine $B_2$ (riboflavine, phosphate de riboflavine), la vitamine $B_6$ (pyridoxine, pyridoxal, pyridoxamine, 5'-phosphate de pyridoxal), la lysine, la méthionine, la cystine, la choline, l'inositol.

**6.** Procédé selon l'une quelconque revendication précédente, dans lequel le microorganisme est sélectionné parmi *Lactobacillus delbrueckii subsp. Lactis, Enterococcus hirae; Lactobacillus plantarum, Lactobacillus fermenti, Lactobacillus helveticus, L. veridescenc, Streptococcus faecalis.*

**7.** Procédé selon l'une quelconque revendication précédente, dans lequel le vaisseau de culture est un plateau à micro-titration.

**8.** Appareil d'essai pour la détermination quantitative microbiologique de vitamines individuelles, d'acides aminés et d'autres composés essentiels à la vie dans un mélange de composés, comprenant des vaisseaux de culture pour la détermination microbiologique de croissance et de métabolisme, où les vaisseaux de culture sont les cavités d'un plateau à micro-titration, qui chacune comprennent un nombre pré-déterminé de cellules vitales d'un microorganisme approprié, qui comprennent par addition d'une solution de gel 200 à 500 mmol/L tréhalose, et qui sont rendues durables pour un stockage sec à température ambiante par gel en choc et lyophilisation.

**9.** Appareil d'essai selon la revendication 8, dans lequel le microorganisme est sélectionné parmi *Lactobacillus delbrueckii subsp. Lactis, Enterococcus hirae; Lactobacillus plantarum, Lactobacillus fermenti, Lactobacillus helveticus, L. veridescenc, Streptococcus faecalis.*

## Fig. 1

### Niacin-Kalibration

## Fig. 2

### Folsäure-Standardkurve

## Fig. 3

**Vitamin B12-Kalibration**

## Fig. 4

**Biotin-Kalibration**

## Fig. 5

**Vitamin B6-Kalibration**

## Fig. 6

**Kalibration Ca-Pantothenat**

# Fig. 7

**Riboflavin-Standardkurve**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6610531 B
- US 5149653 B

**In der Beschreibung aufgeführte Nicht-Patentliteratur**

- **GORIN,G. et al.** *Appl. Microbiol.,* 1970, vol. 20, 641-642
- **KELLEHER,BP et al.** *J. Clin. Pathol.,* 1991, vol. 44 (7), 592-595
- **BUI,M.H.** *J. Vitam. Nutr. Res.,* 1999, vol. 69 (5), 362-366
- **MOLOY,A.M. et al.** *Methods Enzymol.,* 1997, vol. 281, 43-53
- **CONRAD,PB et al.** *Cryobiology,* 2000, vol. 41, 17-24